# EUROPEAN PATENT SPECIFICATION

(11) **EP 2 558 077 B1**
(45) Date of publication and mention of the grant of the patent: **01.03.2017**
(21) Application number: 11769045.3
(22) Date of filing: 12.04.2011
(51) Int. Cl.: A61K 9/22, A61K 9/16, A61K 9/50, A61K 31/192

(54) **ORAL PHARMACEUTICAL COMPOSITION COMPRISING FENOFIBRIC ACID AND AN ALKALIFYING AGENT**
ORAL VERABREICHTE PHARMAZEUTISCHE ZUSAMMENSETZUNG MIT FENOFIBRINSÄURE UND EINEM ALKALISIERUNGSMITTEL
COMPOSITION PHARMACEUTIQUE ORALE COMPRENANT DE L'ACIDE FÉNOFIBRIQUE ET UN AGENT ALCALINISANT

(30) Priority: 12.04.2010 KR 20100033497
(43) Date of publication of application: 20.02.2013
(73) Proprietor: Hanmi Science Co., Ltd., Gyeonggi-do 445-813 (KR)
(72) Inventor: JANG, Ki Young, Suwon-si Gyeonggi-do 440-330 (KR); PARK, Mijin, Hwaseong-si Gyeonggi-do 445-721 (KR); KIM, Kyeong Soo, Suwon-si Gyeonggi-do 442-150 (KR); KIM, Yong Il, Suwon-si Gyeonggi-do 442-718 (KR); PARK, Jae Hyun, Suwon-si Gyeonggi-do 443-470 (KR); WOO, Jong Soo, Suwon-si Gyeonggi-do 440-710 (KR)
(74) Representative: Goddar, Heinz J.
(86) International application number: PCT/KR2011/002560
(87) International publication number: WO 2011/129579

(56) References cited:
- US-A- 4 800 079
- US-A- 4 961 890
- US-A1- 2001 006 662
- US-A1- 2006 280 791
- US-A1- 2007 014 846
- US-A1- 2007 071 812
- US-A1- 2007 148 233
- US-A1- 2008 051 411
- US-A1- 2009 035 379
- US-A1- 2009 263 477
- US-B1- 6 531 158
- US-B1- 7 569 612

## Description

### Technical Field

The present invention relates to an oral pharmaceutical composition comprising fenofibric acid or a pharmaceutically acceptable salt thereof and an alkalifying agent, which has improved bioavailability and a minimized absorption deviation in the gastrointestinal tract.

### Background Art

Fenofibrate can be used to treat intrinsic hyperlipidemia, hypercholesterolemia, and hypertriglyceridemia. It has been known that a daily dose of 300 to 400 mg fenofibrate can reduce the levels of hypercholesterolemia by 20∼25% and hypertriglyceridemia by 40∼50%.

Fenofibrate is metabolized in plasma to the active metabolite, fenofibric acid (chemical name: 2-[4-(4-chlorobenzoyl)phenoxy]-2-methyl-propanoic acid). Fenofibric acid in plasma is eliminated with a half-life of about 20 hours, and peak plasma levels of fenofibric acid occur in about 5 hours after administration.

Fenofibric acid is known to lower levels of total cholesterol (total-C), low density lipoprotein (LDL-C), apo-lipoprotein B, total triglyceride- and triglyceride-rich lipoprotein (VLDL), high density lipoprotein (HDL), and apo-lipoprotein AI and AII in treated patients.

As fenofibrate and fenofibric acid are hydrophobic and poorly soluble in water, they have low bioavailability and their absorption in the digestive tract increases when administered shortly after a meal (fed conditions), as compared to administration under fasting conditions. In general, retention times in the gastrointestinal tract become much longer in fed conditions. As such, when bioavailability of a drug is influenced by the presence of food in the gastrointestinal tract, it is regarded that the drug exhibits food effect. In case of fenofibrate, as food can increase bioavailability of fenofibrate, failure to take fenofibrate with food may lead to significantly decreased absorption. A commercially available fenofibrate-containing product, Tricor® (Abbot), shows an increased absorption rate up to about 35% in fed conditions, compared to administration under fasting conditions. Meanwhile, fenofibric acid has higher solubility at higher pH in the small intestine.

For the reason, a variety of approaches have been attempted to increase the dissolution rate of fenofibrate or to minimize the food effect (e.g., micronization of fenofibrate, addition of surfactants, co-micronization fenofibrate with surfactants).

For example, European Patent No. 256933 describes micronized fenofibrate granules having improved fenofibrate bioavailability. In the granule, the size of crystalline fenofibrate microparticles is smaller than 50 µm and polyvinylpyrrolidone is used as a binder. The European patent also suggests a different type of binders such as methacrylic polymers, cellulose derivatives, and polyethylene glycol. Alternatively, European Patent No. 245933 discloses the preparation of fenofibrate granules using an organic solvent.

European Patent No. 330532 discloses a method for improving fenofibrate bioavailability by co-micronizing fenofibrate with a solid surfactant such as sodium lauryl sulfate. The co-micronized product is formulated into granules by wet granulation in order to enhance the fluidity of powder and make its formulation into gelatin capsules easier.

International Publication No. WO 98/31361 suggests a fenofibrate composition having improved bioavailability, which comprises an inert water-dispersable support coated with a film containing micronized fenofibrate, a hydrophilic polymer (e.g., polyvinylpyrrolidone), and optionally a surfactant. However, as the method requires a substantial amount of polyvinylpyrrolidone and other excipients, only a formulation containing fenofibrate in a small amount of 17.7 wt% can be obtained. Hence, the volume of the final dosage form becomes too large, and thus it is difficult to administer a desired dose of fenofibrate in a sole or complex formulation.

Meanwhile, US Patent No. 7259186 discloses a salt of fenofibric acid selected from the group consisting of choline, ethanolamine, diethanolamine, piperazine, calcium, and tromethamine salt, in order to reduce the food effect on bioavailability. US Patent No. 152714 also describes a preparation of choline salt of fenofibric acid in working examples and shows that the food effect was reduced by the ionic strength of the salt.

US 2008/051411 A1 is related to a formulation in the form of a molecular dispersion comprising i) fenofibric acid, a physiologically acceptable salt or derivative thereof and optionally other active substances, ii) a binder component comprising at least one enteric binder, and optionally iii) other physiologically acceptable excipients.

US 7,569,612 B1 discloses a fenofibric acid formulation comprising 105 mg of fenofibric acid and respective dosage forms including, for example, immediate-release dosage forms.

US 2007/148233 A1 provides a pharmaceutical composition of fenofribrate and dosage forms containing them that include fenofribrate, a polyethylene glycol and a polyethylene-polypropylene glycol, wherein the compositions are made by subliming or sublimable carrier from a combination of fenofibrate, the polyethylene glycol, and the polyethylene-polypropylene glycol with the sublimable carrier, for example menthol.

US 2006/280791 A1 relates to oral formulations comprising an active agent which may comprise fenofibric acid or derivatives thereof.

US 2007/014846 A1 discloses pharmaceutical compositions in particulate form or in solid dosage forms comprising combination of fenofibrate and the HMG CoA reductase inhibitor atovastatin or a pharmaceutically active salt thereof, which upon oral administration provides a relative AUC0-24 value.

As noted above, there have been continuous needs for development of a pharmaceutical composition containing fenofibric acid, which has improved water-solubility and high bioavailability with lower food effect, thereby having a minimized absorption deviation in the gastrointestinal tract.

### Disclosure of Invention

It is an object of the present invention to provide an oral pharmaceutical composition comprising fenofibric acid which has improved bioavailability and a minimized absorption deviation in the gastrointestinal tract.

This object is achieved by the subject-matter of the independent claims. Preferred embodiments result from the sub-claims.

In accordance with one aspect of the present invention, there is provided an oral pharmaceutical composition comprising fenofibric acid or a pharmaceutically acceptable salt thereof and an alkalifying agent.

### Brief Description of the Drawings

The above and other objects and features of the present invention will become apparent from the following description of the invention, when taken in conjunction with the accompanying drawings, which respectively show:
Fig. 1: water-solubilities (*µ*g/ml) of fenofibrate, fenofibric acid and choline fenofibrate, as measured in Test Example 1;
Fig. 2: levels (*µ*g/ml) in the blood of rats of fenofibrate, fenofibric acid and choline fenofibrate, as measured in Test Example 2;
Fig. 3: change in the length ratio (aspect ratio) of the major axis / minor axis of the pellet by variation in the weight ratio of the alkalifying agent and fenofibric acid, as observed in Test Example 4;
Fig. 4: dissolution profiles by pH variation of the fenofibric acid pellets and tablets prepared in Examples 13 and 14, and Comparative Examples 10, 12 and 13, as observed in Test Example 5;
Figs. 5 and 6: dissolution profiles by paddle rotation speed variation of the fenofibric acid pellets and tablets prepared in Example 14 and Comparative Example 12, respectively, as measured in Test Example 6; and
Fig. 7: levels (*µ*g/ml) in the blood of beagles of the fenofibric acid pellets prepared in Comparative Example 10, and Examples 13 and 14, as measured in Test Example 7.

### Best Mode for Carrying Out the Invention

The oral pharmaceutical composition according to the present invention comprises fenofibric acid or a pharmaceutically acceptable salt thereof, and 0.22 to 1 part by weight of an alkalifying agent based on 1 part by weight of fenofibric acid. The alkalifying agent directly contacts with fenofibric acid or a pharmaceutically acceptable salt thereof, which can increase micro-environmental pH around fenofibric acid upon exposed to the *in vivo* environment, thereby increasing water-solubility and consequently bioavailability of fenofibric acid.

The oral pharmaceutical composition according to the present invention is formulated in the form of a pellet which can be filled into a capsule. The pellet may be relatively easily prepared as a coating formulation by coating with a delayed-release coating substrate. Also, the pellet is apt to be simply formulated according to a drug content to be required due to its convenient content control. Especially, in order to minimize *the in vivo* release deviation of a drug and maintain a constant release rate thereof, the pellet is required to have the form close to a spherical form by way of making the length of the major axis similar to that of the minor axis. For example, the pellet is of a form having, i.e., a length ratio ranging from 1.0 to 1.5, preferably 1.0 to 1.2. Therefore, the inventive pharmaceutical composition further comprises a spheronizing additive.

The pellet form of the pharmaceutical composition of the present invention may be prepared by the method comprising the steps of:
(i) first wet-mixing fenofibric acid or a pharmaceutically acceptable salt thereof and an alkalifying agent;
(ii) adding a pharmaceutically acceptable excipient to the first mixture obtained in step (i), and second wet-mixing them; and
(iii) granulating the second mixture obtained in step (ii) into a spherical form using an extruder and a spheronizer.

Optionally, in step (ii), the method may comprise further adding a spheronizing additive to the first mixture. In addition, the method may further comprise coating the granule obtained in step (iii) with a delayed-release coating substrate.

Each of ingredients of the inventive oral pharmaceutical composition is described in detail as follows.

### (1) Fenofibric acid or a pharmaceutically acceptable salt thereof

Fenofibric acid or its pharmaceutically acceptable salt used as an active ingredient in the present invention is a fenofibrate metabolite, which attributes to substantially decrease a level of plasma triglyceride in a patient suffering from hypertriglyceridemia, and levels of plasma cholesterol and LDL-C in a patient suffering from hypertriglyceridemia or mixed-type hyperlipidemia.

### (2) Alkalifying agent

The alkalifying agent used in the present invention is an additive for improving solubility of fenofibric acid or its pharmaceutically acceptable salt. The alkalifying agent directly contacts with fenofibric acid or a pharmaceutically acceptable salt thereof, which can increase micro-environmental pH around fenofibric acid upon exposed to *the in vivo* environment, thereby increasing water-solubility and consequently bioavailability of fenofibric acid. Also, the alkalifying agent increases solubility of fenofibric acid or its pharmaceutically acceptable salt in a low pH environment.

Representative examples of the alkalifying agent used in the present invention include alkali metal salts such as calcium salts (calcium carbonate, calcium hydroxide, calcium hydrogen phosphate, calcium phosphate), magnesium salts (magnesium carbonate, magnesium hydroxide, magnesium silicate, magnesium oxide, magnesium aluminate, magnesium aluminum hydrate), lithium salts (lithium hydroxide), potassium salts (potassium hydroxide), sodium phosphate (sodium bicarbonate, sodium borate, sodium carbonate, sodium hydroxide) and the like. Also, a basic additive such as meglumine, arginine and a mixture thereof may be used. In accordance with the invention, the alkalifying agent is calcium carbonate, magnesium carbonate, meglumine, or a mixture thereof.

The alkalifying agent is employed in an amount ranging from 0.22 to 1 part by weight based on 1 part by weight of the fenofibric acid or a pharmaceutically acceptable salt. When the amount of the alkalifying agent is less than 0.22 parts by weight, the water-solubility of fenofibric acid cannot be achieved to the desired level of at least 1000 ppm; and when more than 1 part by weight, the length ratio of the major axis and the minor axis of the pellet exceeds 1.5, which results in a difficulty in the pellet spheronization, thereby leading to formation of a pellet unsuitable for the coating process.

### (3) Delayed-release coating substrate

The fenofibric acid pellet containing the alkalifying agent according to the present invention may be coated with a delayed-release coating substrate. The coating with such a delayed-release coating substrate helps the pellet release a drug at a constant rate until the pellet reaches a high pH region, which brings out a minimized absorption deviation in the gastrointestinal tract.

The delayed-release coating substrate used in the present invention includes a water-insoluble polymer or hydrophobic compound.

The water-insoluble polymer may be selected from the group consisting of hydroxypropylmethylcellulose phthalate (HPMCP), polyvinyl acetate (e.g., KOLLICOAT SR 30D), water-insoluble polymethacrylate copolymers [e.g., poly(ethylacrylate-methylmethacrylate) copolymers (e.g., Eudragit NE30D), poly(ethylacrylate-methylmethacrylate-trimethylaminoethylmethacrylate chloride) copolymers (e.g., Eudragit RSPO) and the like], ethylcellulose, cellulose ester, cellulose ether, cellulose acylate, cellulose diacylate, cellulose triacylate, cellulose acetate, cellulose diacetate, cellulose triacetate, and a mixture thereof; preferably at least one selected from the group consisting of hydroxypropylmethylcellulose phthalate (HPMCP), polyvinyl acetate, poly(ethylacrylate-methylmethacrylate) copolymers, poly(ethylacrylate-methylmethacrylate-trimethylaminoethylmethacrylate chloride) copolymers, ethylcellulose, and cellulose acetate; more preferably at least one selected from the group consisting of hydroxypropylmethylcellulose phthalate (HPMCP), poly(ethylacrylate-methylmethacrylate) copolymers, ethylcellulose, and cellulose acetate.

The hydrophobic compound may be selected from the group consisting of fatty acid, fatty acid esters, fatty acid alcohols, waxes, an inorganic material, and a mixture thereof. The fatty acid and fatty acid esters may be at least one selected from the group consisting of glyceryl palmitostearate, glyceryl stearate, glyceryl behenate, cetyl palmitate, glyceryl monooleate, and stearic acid; the fatty acid alcohols, at least one selected from the group consisting of cetostearyl alcohol, cetyl alcohol, and stearyl alcohol; the waxes, at least one selected from the group consisting of carnauba wax, beeswax, and micro-crystalline waxes; and the inorganic material, at least one selected from the group consisting of talc, precipitated calcium carbonate, dicalcium phosphate, zinc oxide, titanium oxide, kaolin, bentonite, montmorillonite, and veegum. The hydrophobic compound may be preferably at least one selected from the group consisting of glyceryl palmitostearate, glyceryl behenate, stearic acid, cetyl alcohol and carnauba wax, more preferably, at least one selected from the group consisting of glyceryl behenate, stearic acid, and carnauba wax.

The delayed-release coating substrate may be employed in an amount ranging from 0.05 to 0.5 parts by weight based on 1 part by weight of the pellet (the pellet before coated) as a core. When the amount of the delayed-release coating substrate is less than 0.05 parts by weight, no desired delayed-release effects is obtained; and when more than 0.5 parts by weight, the size of the coated pellet becomes too large, thereby causing formation of too big sized capsule.

### (4) Spheronizing additive

The spheronizing additive used in the present invention serves to prepare the pellet which has the form close to a spherical form by controlling the length of the major axis similar to that of the minor axis and to give a constant mechanical coherence thereto. The spheronizing additive includes a natural macromolecule such as carrageenan gum (in accordance with the invention), guar gum (in accordance with the invention), xanthane gum, locust bean gum, gellan gum, arabia gum, agar, alginic acid, alginic acid propylene glycol (in accordance with the invention), sodium alginate, and a mixture thereof.

The spheronizing additive may be employed in an amount ranging from 0.05 to 0.5% by weight based on the total weight of the pellet (the pellet before coated) corresponding to a core. When the spheronizing additive is employed in an amount of less than 0.05% by weight, it is difficult to obtain the desired spheronizing effect; and when in an amount of more than 0.5% by weight, delay of release undesirably occurs or the pellet can be adherent to the production equipments due to its adhesive property in an extrusion process.

Also, the inventive pharmaceutical composition may further comprise a suitable amount of a pharmaceutically acceptable excipient such as a conventional disintegrating agent, a diluent, a stabilizer, a binder, a lubricating agent and the like.

The inventive pharmaceutical composition may be orally administered in a pellet form, and the preparation method thereof comprises the steps of: (i) first wet-mixing fenofibric acid or a pharmaceutically acceptable salt thereof and an alkalifying agent; (ii) adding a pharmaceutically acceptable excipient to the first mixture obtained in step (i), and second wet-mixing them; and (iii) granulating the second mixture obtained in step (ii) into a spherical form using an extruder and a spheronizer.

Optionally, in step (ii), the method may comprise further adding a spheronizing additive to the first mixture. In addition, the method may further comprise coating the granule obtained in step (iii) with a delayed-release coating substrate.

The inventive oral pharmaceutical composition comprising fenofibric acid exhibits improved bioavailability and a minimized absorption deviation in the gastrointestinal tract, and, accordingly, it is useful in treating hyperlipidemia and hypertriglyceridemia.

The following Examples are intended to further illustrate the present invention without limiting its scope.

### Test Example 1: Solubility Test of fenofibric acid, fenofibrate, and choline fenofibrate

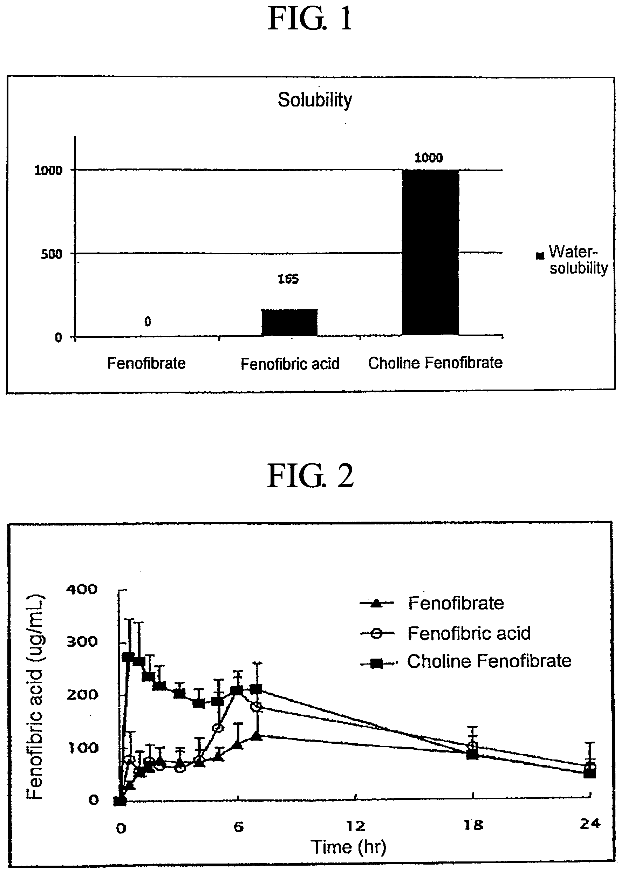

Each of fenofibrate (Harman, India), fenofibric acid (Harman, India) and choline fenofibrate was placed in the amount equivalent to 100 mg fenofibric acid in a 100 mL flask. Each of distilled water and artificial intestinal juice (pH 6.8; USP) was added thereto to fill 100 mL, and the mixture was vigorously blended for 1 hr and passed through a 0.45 µm filter, followed by HPLC analysis. The solubilities (ug/ml) of the compounds are shown in Table 1 and Fig. 1 (analysis method: *see* 'Fenofibrate' in USP).

**<Table 1>**

| | Fenofibrate | Fenofibric acid | Choline fenofibrate |
|---|---|---|---|
| Distilled water | 0 | 165 | 1000 |
| Artificial intestinal juice (pH 6.8) | 0 | 1000 | 1000 |

As can be seen in Table 1 and Fig. 1, fenofibrate was nearly insoluble in water or other solvents, while the solubility in water of fenofibric acid was higher than that of fenofibrate, but lower than that of choline fenofibrate, showing that the water-solubilites of above compounds are in an order of choline fenofibrate > fenofibric acid > fenofibrate. Meanwhile, fenofibric acid and choline fenofibrate were completely dissolved at pH 6.8, as evidenced by their solubility of 1000 ppm.

For reference, it is recommended to take a drug with 200 to 300 mL of water, and more preferably 240 mL according to the Korean Guideline for Bioequivalence Studies. In addition, considering that the volume of gastric juice in fasting conditions is about 45 mL and the stomach expands up to 1000 mL in fed conditions (Sherwood, Lauralee (1997). *Human physiology: from cells to systems.* Belmont, CA: Wadsworth Pub. Co), *high in vivo* bioavailability of fenofibrate would be expected when water-solubility of the main component becomes not less than 1000 ppm, such that 200 mg of fenofibrate present in a fenofibrate-containing capsule (e.g., Lipanthyl®) can be sufficiently dissolved when being taken with 200 mL of water in fasting conditions.

### Test Example 2: Pharmacokinetic evaluation of raw materials

Each of fenofibrate, fenofibric acid and choline fenofibrate was placed in the amount equivalent to 88mg fenofibric acid into microcapsules. The microcapsules were orally administered to 7 week-old male SD rats (Sprague Dawley, Samtako, 4 rats per one group, 7 days feeding *ad libitum*) by using Sonde. Blood samples were taken from the rats in a predetermined time interval, and the respective pharmacokinetic profiles were evaluated. The results are shown in Table 2 and Fig. 2.

**<Table 2>**

| | Fenofibrate | Fenofibric acid | Choline fenofibrate |
|---|---|---|---|
| AUC 0-24 (µg·hr/mL) | 2078.3 ± 610.5 | 2742.2 ± 218.1 | 3477.7 ± 558.9 |
| Cmax (µg/mL) | 137.2 ± 37.9 | 209.3 ± 26.1 | 309.4 ± 62.2 |

As evidenced by Table 2 and Fig.2, the absorption rates were shown to be in an order of choline fenofibrate > fenofibric acid > fenofibrate, which were identical to the solubilities in water obtained in Test Example 1, showing the correlation between the water-solubility and the absorption rate of a drug.

### Examples 1 to 9 and Comparative Examples 1 to 9: Preparation of fenofibric acid pellets comprising alkalifying agents

In order to increase micro-environmental pH around fenofibric acid, fenofibric acid and an alkalifying agent were added in an amount as set forth in Table 3 to a certain amount of water and mixed thoroughly (the first wet-mixing). Then, povidone (BASF, Germany) was added thereto and mixed thoroughly (the second wet-mixing) to obtain a combined mixture. The mixture was extruded through a 0.8 mm-mesh sieve using an extruder (MG-55, Dalton) and spheronized for 3 min using a spheronizer (Spheronizer Q-230T, Dalton), followed by drying at 60°C to obtain pellets.

**<Table 3>**

| **(unit: mg)** | | | | | | |
|---|---|---|---|---|---|---|
| | **Fenofibric acid** | **Alkalifying agent** | | | **Povidone** | **Weight ratio of alkalifying agent/ fenofibric acid** |
| | | **Meglumine** | **Magnesium carbonate** | **CaCO₃** | | |
| Comp. Ex. 1 | 135 | 10 | | | 35 | 0.07 |
| Comp. Ex. 2 | 135 | 20 | | | 35 | 0.15 |
| Ex. 1 | 135 | 30 | | | 35 | 0.22 |
| Ex. 2 | 135 | 80 | | | 35 | 0.59 |
| Ex. 3 | 135 | 135 | | | 35 | 1.00 |
| Comp. Ex. 3 | 135 | 200 | | | 35 | 1.48 |
| Comp. Ex. 4 | 135 | | 10 | | 35 | 0.07 |
| Comp. Ex. 5 | 135 | | 20 | | 35 | 0.15 |
| Ex. 4 | 135 | | 30 | | 35 | 0.22 |
| Ex. 5 | 135 | | 80 | | 35 | 0.59 |
| Ex. 6 | 135 | | 135 | | 35 | 1.00 |
| Comp. Ex. 6 | 135 | | 200 | | 35 | 1.48 |
| Comp. Ex. 7 | 135 | | | 10 | 35 | 0.07 |
| Comp. Ex. 8 | 135 | | | 20 | 35 | 0.15 |
| Ex. 7 | 135 | | | 30 | 35 | 0.22 |
| Ex. 8 | 135 | | | 80 | | 0.59 |
| Ex. 9 | 135 | | | 135 | | 1.00 |
| Comp. Ex. 9 | 135 | | | 200 | | 1.48 |

### Comparative Examples 10 and 11: Preparation of fenofibric acid pellets containing no alkalifying agent

The procedure of Example 1 was repeated based on the conditions as set forth in Table 4 below, except for using no alkalifying agent.

**<Table 4>**

| (unit: mg) | | | | |
|---|---|---|---|---|
| | Fenofibric acid | Choline Fenofibrate | Povidone | Weight ratio of alkalifying agent/ fenofibric acid |
| Comp. Ex. 10 | 135 | | 35 | 0.00 |
| Comp. Ex. 11 | | 178.7 (135 for fenofibric acid) | 35 | 0.00 |

### Examples 10 to 12 : Preparation of fenofibric acid pellets comprising spheronizing additives

The procedure of Example 1 was repeated based on the conditions as set forth in Table 5, except for adding carageenan gum (FMC biopolymer), guar gum (Haji dossa) or propylene glycol alginate (ISP) as a spheronizing additive on the second wet-mixing.

**<Table 5>**

| (unit: mg) | | | | | |
|---|---|---|---|---|---|
| | Fenofibric acid | Spheronizing additive | | | Magnesium carbonate |
| | | carageenan gum | guar gum | propylene glycol alginate | |
| Ex. 10 | 135 | 35 | | | 135 |
| Ex. 11 | 135 | | 35 | | 135 |
| Ex. 12 | 135 | | | 35 | 135 |

### Test Example 3 : Comparison of solubilities of fenofibric acid according to the amount of alkalifying agents

The water-solubilities (µg/mL) of fenofibric acid were measured by employing the pellets prepared in Examples 1 to 9 and Comparative Examples 1 to 11 in amounts equivalent to 100 mg fenofibric acid, in accordance with the procedure of Test Example 1. The results are shown in Table 6.

**<Table 6>**

| | Weight ratio of alkalifying agent/fenofibric acid | Water-solubility |
|---|---|---|
| Comp. Ex. 1 | 0.07 | 478 |
| Comp. Ex. 2 | 0.15 | 740 |
| Ex. 1 | 0.22 | 1001 |
| Ex. 2 | 0.59 | 1000 |
| Ex. 3 | 1.00 | 1002 |
| Comp. Ex. 3 | 1.48 | 1001 |
| Comp. Ex. 4 | 0.07 | 465 |
| Comp. Ex. 5 | 0.15 | 733 |
| Ex. 4 | 0.22 | 1001 |
| Ex. 5 | 0.59 | 1002 |
| Ex. 6 | 1.00 | 1003 |
| Comp. Ex. 6 | 1.48 | 1002 |
| Comp. Ex. 7 | 0.07 | 452 |
| Comp. Ex. 8 | 0.15 | 727 |
| Ex. 7 | 0.22 | 1002 |
| Ex. 8 | 0.59 | 1001 |
| Ex. 9 | 1.00 | 1002 |
| Comp. Ex. 9 | 1.48 | 1002 |
| Comp. Ex. 10 | 0.00 | 164 |
| Comp. Ex. 11 | 0.00 | 1000 |

As can be seen in Table 6, the solubility of fenofibric acid increases as the amount of the alkalifying agent increases. When the weight ratio of alkalifying agent/fenofibric acid is not less than 0.22, the water-solubility of the pellet exceeded the targeted level of the present invention, i.e., 1000 ppm. Ths result indicates that the pellets of the present invention have the water-solubility of fenofibric acid equivalent to choline fenofibrate, and would have an *enhanced in vivo* absorption rate.

### Test Examnle 4 : Evaluation of spheronizing degrees

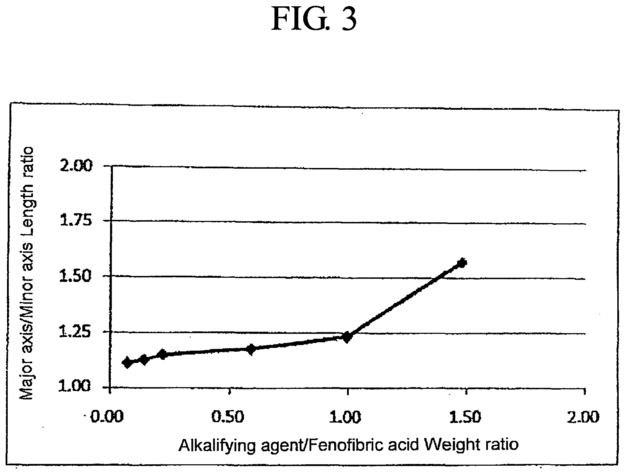

The length ratios of the major axis and the minor axis (aspect ratios) were measured on the pellets prepared in Examples 1 to 12 and Comparative Examples 1 to 9. The measurement results are shown in Table 7 and Fig. 3.

**<Table 7>**

| | Alkalifying agent / Fenofibric acid (w/w) | Aspect ratio |
|---|---|---|
| Comparative Example 1 | **0.07** | 1.13 |
| Comparative Example 2 | **0.15** | 1.21 |
| Example 1 | **0.22** | 1.29 |
| Example 2 | **0.59** | 1.32 |
| Example 3 | **1.00** | 1.45 |
| Comparative Example 3 | **1.48** | 1.69 |
| Comparative Example 4 | **0.07** | 1.11 |
| Comparative Example 5 | **0.15** | 1.13 |
| Example 4 | **0.22** | 1.15 |
| Example 5 | **0.59** | 1.18 |
| Example 6 | **1.00** | 1.23 |
| Comparative Example 6 | **1.48** | 1.57 |
| Comparative Example 7 | **0.07** | 1.17 |
| Comparative Example 8 | **0.15** | 1.19 |
| Example 7 | **0.22** | 1.25 |
| Example 8 | **0.59** | 1.26 |
| Example 9 | **1.00** | 1.32 |
| Comparative Example 9 | **1.48** | 1.56 |
| Example 10 | **1.00** | 1.18 |
| Example 11 | **1.00** | 1.19 |
| Example 12 | **1.00** | 1.22 |

The aspect ratio is an indicator for evaluating a spheronizing degree. The aspect ratio close to 1 means a nearly spherical shape. Generally, when the shape of the pellet is near to a sphere, a coating operation such as delayed-release coating is reproducibly performed, and a mass deviation generated on filling a capsule decreases, thereby resulting in an improvement in productivity (see [Chopra R. et al, Pharm. Dev. Technol. 2002 Jan; 7(1):59-68] and [Chopra R et al, Eur. J. Pharm. Biopharm. 2002 May; 53(3):327-33]). In contrast, poor spheronization deteriorates the coating efficiency. In particular, when a functional coating such as a delayed-release coating is conducted, a coating deviation occurs, which makes it difficult to reproducibly produce the preparation. An aspect ratio ranging from 1.0 to 1.2 are most preferred, while the pellet having an aspect ratio exceeding 1.5 should be reformed.

It can be seen from the results shown in Table 7 and Fig. 3 that the aspect ratio increased in proportion to the amount of the alkalifying agent. Particularly, the pellets obtained in Comparative Examples 3, 6 and 9 comprising the alkalifying agent in an amount of 1.48 times of that of fenofibric acid showed aspect ratios of 1.5 or more and, accordingly, they are inappropriate for coating. However, the pellets of Examples 10 to 12 using a natural polymer spheronizing additive such as carrageenan gum, guar gum and propylene glycol alginate showed improved spheronizing degrees.

Accordingly, it was demonstrated that the most preferable amount of the alkalifying agent is in a range of 0.22 to 1 part by weight based on 1 part by weight of fenofibric acid.

### Example 13: Preparation of fenofibric acid pellets comprising alkalifying agents

The procedure of Example 10 was repeated except that the alkalifying agent (magnesium carbonate) was used in the amount of 50mg, to obtain a pellet.

**<Table 8>**

| (unit: mg) | |
|---|---|
| | Example 13 |
| Fenofibric acid | 135 |
| Carrageenan gum | 35 |
| Magnesium carbonate | 50 |

### Examples 14 to 16: Preparation of fenofibric acid pellets with delayed-release coatings

The pellet obtained in Example 13, as a core, was coated with a delayed-release coating substrate, i.e., hydroxypropylmethylcellulose phthalate (HPMCP, Shinetsu), ethylcellulose (EC, Aqualon) or polyvinyl acetate (PVA, BASF), as shown in Table 9 below. In order to perform a convenient coating, the delayed-release coating substrate was dissolved in a mixture of water and ethanol, and hydroxypropylmethyl cellulose (HPMC, Shinetsu, Japan) and propylene glycol (PEG) were added thereto as coating substrates. The coating process was conducted using a conventional fluidized bed coater, to obtain coated pellets.

**<Table 9>**

| (unit: mg) | | | |
|---|---|---|---|
| | Example 14 | Example 15 | Example 16 |
| Core pellet (pellet of Example 13) | 220 | 220 | 220 |
| EC | 32 | | |
| HPMCP | | 32 | |
| PVA | | | 32 |
| HPMC | 12 | 12 | 12 |
| PEG | 6 | 6 | 6 |
| EtOH | 350 | 350 | 350 |
| Distilled water | 35 | 35 | 35 |

### Comparative Examples 12 and 13: Preparation of fenofibric acid tablet

As shown in Table 10 below, MCC (microcrystalline cellulose, FMC Biopolymer), lactose (DMV pharmaceutical) and/or magnesium stearate as an excipient, and magnesium carbonate as an alkalifying agent were mixed, and the resulting mixture was tableted to obtain tablets comprising fenofibric acid and an alkalifying agent. The tablets were coated using a tablet coater with a coating solution having the same composition as that used in Example 14 (but comprising EC as a delayed-release coating substrate).

**<Table 10>**

| (unit: mg) | | |
|---|---|---|
| | Comparative Example 12 | Comparative Example 13 |
| Fenofibric acid | 105 | 105 |
| Lactose | 100 | 235 |
| MCC | 135 | |
| Magnesium carbonate | 50 | 50 |
| Magnesium stearate | 2 | 2 |
| EC | 32 | 32 |
| HPMC | 12 | 12 |
| PEG | 6 | 6 |
| EtOH | 350 | 350 |
| Distilled water | 35 | 35 |

### Test Example 5: Dissolution test - (1)

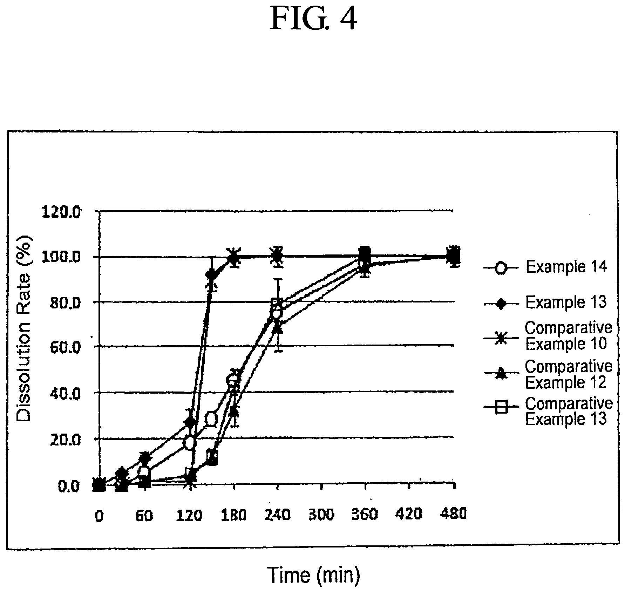

The dissolution rates of the fenofibric acid pellets or tablets obtained in Examples 13 and 14, and Comparative Examples 10, 12 and 13, were tested using the USP paddle II method (rotation rate: 50rpm). The dissolution test was designed considering the retention time in the stomach, i.e., 1 to 2 hours. First, the pellets or tablets were subjected to a dissolution test in 700ml of 0.1N HCl for 2 hours, and then 300ml of a phosphoric acid buffer was added thereto so that the test was continued at pH equal to that of artificial intestinal juice (pH 6.8, USP) (see Dissolution of "delayed-release dosage form" in USP). The results are shown in Table 11 and Fig. 4.

**<Table 11>**

| | Example 14 | | Example 13 | | Comparative Example 10 | | Comparative Example 12 | | Comparative Example 13 | |
|---|---|---|---|---|---|---|---|---|---|---|
| Time (min) | Aver. | S.D. | Aver. | S.D. | Aver. | S.D. | Aver. | S.D. | Aver. | S.D. |
| 0 | 0.0 | 0.0 | 0.0 | 0.0 | 0.0 | 0.0 | 0.0 | 0.0 | 0.0 | 0.0 |
| 30 | 0.0 | 0.0 | 5.2 | 0.2 | 0.0 | 0.0 | 0.0 | 0.0 | 0.0 | 0.0 |
| 60 | 5.0 | 0.2 | 11.2 | 3.3 | 1.1 | 0.0 | 1.1 | 0.1 | 1.2 | 0.1 |
| 120 | 18.2 | 1.8 | 27.3 | 5.2 | 1.2 | 0.0 | 3.8 | 0.6 | 4.2 | 0.6 |
| 150 | 28.3 | 2.8 | 92.3 | 7.5 | 89.0 | 2.7 | 11.4 | 2.7 | 12.3 | 3.0 |
| 180 | 45.2 | 4.5 | 99.2 | 3.5 | 100.2 | 3.5 | 32.4 | 6.5 | 42.3 | 8.5 |
| 240 | 75.3 | 4.0 | 100.1 | 4.2 | 100.1 | 3.2 | 68.6 | 10.3 | 78.9 | 11.8 |
| 360 | 96.5 | 5.2 | 100.2 | 4.4 | 100.2 | 3.4 | 95.4 | 1.3 | 100.2 | 2.0 |
| 480 | 100.0 | 3.0 | 100.2 | 4.5 | 100.2 | 3.0 | 100.2 | 1.5 | 100.2 | 1.8 |

It can be seen from the results shown in Table 11 and Fig. 4 that the pellet obtained in Comparative Example 10, which has neither alkalifying agent nor delayed-release coating, exhibited very low dissolution rate at low pH, but the dissolution rate thereof increased sharply at pH 6.8 due to the relatively high solubility of fenofibric acid. Accordingly, it is predicted that only a part of the drug eluted during staying at the stomach of low pH can be absorbed due to its low solubility, while the absorption of the drug would sharply increase at the small intestine of high pH due to the high solubility of the drug therein. The similar result is observed with the pellet of Example 13 comprising an alkalifying agent, while the solubility of the drug at low pH was significantly improved due to presence of the alkalifying agent. Moreover, with the pellet obtained in Example 14, which further comprises the delayed-release coating, the drug was released slowly at a constant rate, regardless of pH variation. It is expected that such release pattern would minimize the absorption deviation in the gastrointestinal tract *in vivo.*

### Test Example 6: Dissolution test - (2)

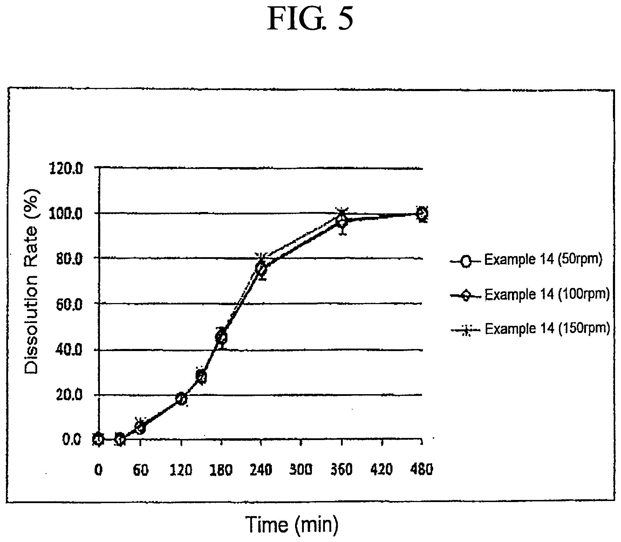

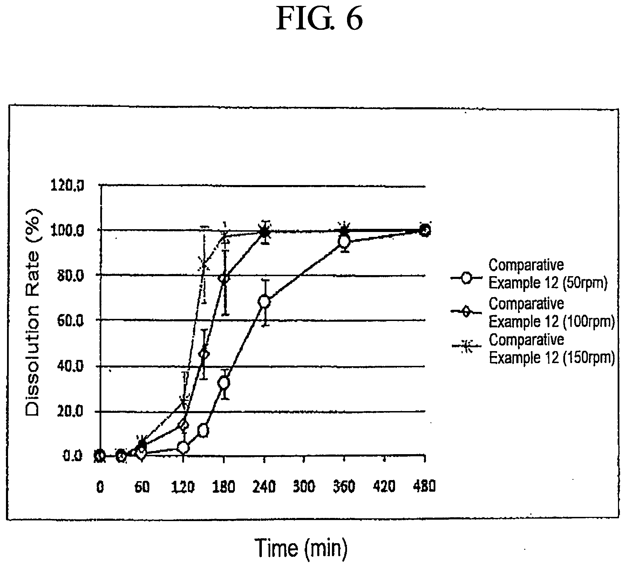

The dissolution rates of the fenofibric acid pellets or tablets obtained in Example 14 and Comparative Example 12 were tested according to the same procedure as in Test Example 5, except for changing the rotation rate of the paddle. The results are shown in Table 12, and Figs. 5 and 6.

**<Table 12>**

| | Example 14 | | | | | | Comparative Example 12 | | | | | |
|---|---|---|---|---|---|---|---|---|---|---|---|---|
| | 50rpm | | 100rpm | | 150rpm | | 50rpm | | 100rpm | | 150rpm | |
| Time (min) | Aver. | S.D. | Aver. | S.D. | Aver. | S.D. | Aver. | S.D. | Aver. | S.D. | Aver. | S.D. |
| 0 | 0.0 | 0.0 | 0.0 | 0.0 | 0.0 | 0.0 | 0.0 | 0.0 | 0.0 | 0.0 | 0.0 | 0.0 |
| 30 | 0.0 | 0.0 | 0.0 | 0.0 | 0.0 | 0.0 | 0.0 | 0.0 | 0.0 | 0.0 | 0.0 | 0.0 |
| 60 | 5.0 | 0.2 | 6.1 | 1.7 | 6.6 | 1.0 | 1.1 | 0.1 | 4.2 | 2.3 | 6.2 | 0.6 |
| 120 | 18.2 | 1.8 | 18.0 | 2.2 | 17.7 | 2.2 | 3.8 | 0.6 | 14.3 | 10.7 | 24.3 | 13.4 |
| 150 | 28.3 | 2.8 | 27.6 | 1.3 | 29.0 | 1.0 | 11.4 | 2.7 | 45.3 | 10.9 | 85.3 | 17.1 |
| 180 | 45.2 | 4.5 | 45.9 | 3.4 | 47.2 | 3.7 | 32.4 | 6.5 | 78.9 | 15.8 | 97.9 | 6.1 |
| 240 | 75.3 | 4.0 | 75.9 | 4.0 | 79.7 | 3.8 | 68.6 | 10.3 | 99.8 | 5.0 | 99.8 | 1.9 |
| 360 | 96.5 | 5.2 | 96.9 | 5.4 | 99.6 | 5.0 | 95.4 | 4.3 | 100.2 | 2.0 | 100.6 | 2.0 |
| 480 | 100.0 | 3.0 | 100.0 | 3.6 | 100.3 | 2.9 | 100.2 | 1.5 | 100.9 | 1.8 | 101.1 | 1.8 |

It can be seen from the results shown in Table 12 and Figs. 5 and 6 that the dissolution rate of the pellet obtained in Example 14 was rarely influenced by the change of the rotation rate of the paddle, while that of the tablet obtained in Comparative Example 12 was strongly affected thereby. In general, the degree of drug release according to the movement degree of an internal organ can be predicted from the variation in the dissolution rates according to the rotation rate of the paddle. Accordingly, it is expected that tablets such as that obtained in Comparative Example 12 would exhibit a large deviation in *the in vivo* dissolution rate. In contrast, pellets such as that obtained in Example 14 are predicted to exhibit a small deviation in the *in vivo* dissolution rate, accordingly, a pellet is considered as a preferable formulation.

### Test Example 7: Pharmacokinetic evaluation

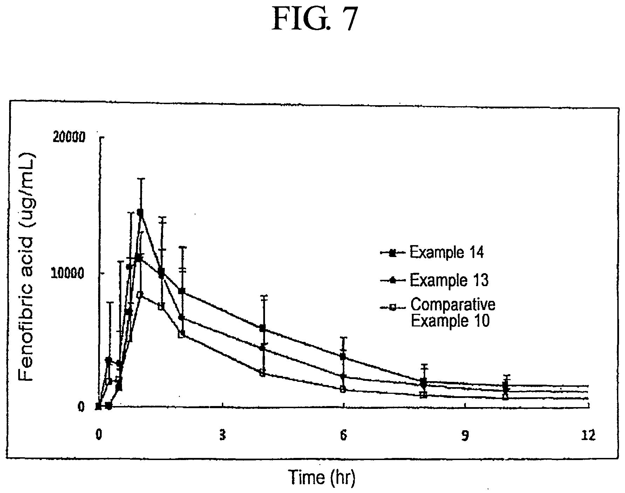

Pharmacokinetic evaluation of the fenofibric acid pellets obtained in Comparative Example 10 and Examples 13 and 14 was conducted in beagles. The pellets were administered to three groups each having four beagles, and blood samples thereof were taken at regular time intervals to measure pharmacokinetic (pK) profiles, and the results are shown in Table 13 and Fig. 7.

**<Table 13>**

| | Example 14 | Example 13 | Comparative Example 10 |
|---|---|---|---|
| AUC 0-24 (ng.hr/ml) | 74366.9 ± 19893 | 641.04.1 ± 27882 | 42565.3 ± 18192 |
| Cmax (ng/ml) | 15642.4 ± 3894 | 13529.1 ± 6998 | 9067.4 ± 4453 |
| AUC (S.D./Aver.) | 27% | 43% | 43% |
| Cmax (S.D./Aver.) | 25% | 52% | 49% |

As shown in Table 13 and Fig. 7, the fenofibric acid pellet obtained in Example 13 comprising an alkalifying agent exhibited the absorption rate higher than that obtained in Comparative Example 10. Meanwhile, the pellet obtained in Example 13 showed the dual pK profiles due to the first absorption in the stomach and the second absorption in the small intestine. In contrast, the pellet obtained in Example 14, which further has a delayed-release coating, exhibits a balanced and high absorption rate. Moreover, from the ratios of the standard deviation (S.D.) to the average value of each of AUC and Cmax, it is understood that the pellet of Example 14 with a delayed-release coating has the small deviation in the absorption rate over the pK stomach and the small intestine. This result demonstrates that the pellet of the present invention would be very useful for treating hyperlipidemia and hypertriglyceridemia.

While the invention has been described with respect to the above specific embodiments, it should be recognized that various modifications and changes may be made to the invention by those skilled in the art which also fall within the scope of the invention as defined by the appended claims.

## Claims

1. An oral pharmaceutical composition comprising fenofibric acid or a pharmaceutically acceptable salt thereof, an alkalifying agent and a spheronizing additive,
wherein the composition is in the form of a pellet;
wherein the alkalifying agent is selected from the group consisting of calcium carbonate, magnesium carbonate, meglumine, and a mixture thereof and
is used in an amount ranging from 0.22 to 1 part by weight based on 1 part by weight of fenofibric acid;
wherein the spheronizing additive is selected from the group consisting of carrageenan gum, guar gum, propylene glycol alginate, and a mixture thereof and is used in an amount ranging from 0.05 to 0.5% by weight based on the total weight of the pellet;
wherein the pellet has a length ratio of the major axis and the minor axis ranging from 1.0 to 1.5; and
wherein the pellet is further coated with a delayed-release coating substrate.

2. The composition of claim 1, wherein the delayed-release coating substrate is a water-insoluble polymer.

3. The composition of claim 2, wherein the delayed-release coating substrate is selected from the group consisting of hydroxypropylmethylcellulose phthalate (HPMCP), ethylcellulose, poly(ethylacrylate-methylmethacrylate) copolymers, cellulose acetate, and a mixture thereof.

4. The composition of claim 1, wherein the delayed-release coating substrate is used in an amount ranging from 0.05 to 0.5 parts by weight based on 1 part by weight of a pellet before coated.

5. A method for preparing the composition of claim 1, which comprises the steps of:
(i) first wet-mixing fenofibric acid or a pharmaceutically acceptable salt thereof and an alkalifying agent;
(ii) adding a pharmaceutically acceptable excipient to the first mixture obtained in step (i), and second wet-mixing them; and
(iii) granulating the second mixture obtained in step (ii) into a spherical form using an extruder and a spheronizer.

6. The method of claim 5, which, in step (ii), comprises further adding a spheronizing additive to the first mixture.

7. The method of claim 5, which further comprises coating the granule obtained in step (iii) with a delayed-release coating substrate.

## Patentansprüche

1. Orale pharmazeutische Zusammensetzung, die Fenobrinsäure oder ein pharmazeutisch akzeptables Salz derselben, ein alkalisch machendes Mittel und ein sphäronisierendes Additiv umfasst,
wobei die Zusammensetzung in Form eines Pellets vorliegt;
wobei das alkalisch machende Mittel ausgewählt ist aus der Gruppe, bestehend aus Calciumcarbonat, Magnesiumcarbonat, Meglumin und einem Gemisch derselben und in einer Menge von 0,22 bis 1 Gewichtsteil, bezogen auf 1 Gewichtsteil der Fenobrinsäure, verwendet wird;
wobei das sphäronisierende Additiv ausgewählt ist aus der Gruppe, bestehend aus Carrageenan, Guarkernmehl, Propylenglykolalginat und einem Gemisch derselben, und in einer Menge von 0,05 bis 0,5 Gew.-%, bezogen auf ein Gesamtgewicht des Pellets, verwendet wird;
wobei das Pellet ein Längenverhältnis der Hauptachse zu der Nebenachse in einem Bereich von 1,0 bis 1,5 hat, und
wobei das Pellet ferner beschichtet ist mit einem freisetzungsverzögernden Beschichtungsträger.

2. Zusammensetzung nach Anspruch 1, wobei der freisetzungsverzögernde Beschichtungsträger ein wasserunlösliches Polymer ist.

3. Zusammensetzung nach Anspruch 2, wobei der freisetzungsverzögernde Beschichtungsträger ausgewählt ist aus der Gruppe, bestehend aus Hydroxypropylmethylcellulosephthalat (HPMCP), Ethylcellulose, Poly(ethylacrylat-methylmethacrylat)copolymeren, Celluloseacetat und einem Gemisch derselben.

4. Zusammensetzung nach Anspruch 1, wobei der freisetzungsverzögernde Beschichtungsträger in einer Menge in einem Bereich von 0,05 bis 0,5 Gewichtsteilen, bezogen auf 1 Gewichtsteil des Pellets vor der Beschichtung, verwendet wird.

5. Verfahren zur Herstellung einer Zusammensetzung nach Anspruch 1, das die folgenden Schritte umfasst:
(i) zunächst Nassmischen von Fenobrinsäure oder einem pharmazeutisch akzeptablen Salz derselben mit einem alkalisch machenden Mittel;
(ii) Zugeben eines pharmazeutisch akzeptablen Hilfsstoffs zu dem ersten Gemisch, das in Schritt (i) erhalten wird, und ein zweites Nassmischen derselben; und
(iii) Granulieren des zweiten Gemischs, das in Schritt (ii) erhalten wurde, in eine sphärische Form unter Verwendung eines Extruders oder eines Sphäronisators.

6. Verfahren nach Anspruch 5, das, in Schritt (ii), ferner das Hinzufügen eines sphäronisierenden Additivs zu dem ersten Gemisch umfasst.

7. Verfahren nach Anspruch 5, das ferner das Beschichten der Körnchen, die in Schritt (iii) erhalten werden, mit einem freisetzungsverzögernden Beschichtungsträger umfasst.

## Revendications

1. Une composition pharmaceutique orale comprenant de l'acide fénofibrique ou un sel pharmaceutiquement acceptable de celui-ci, un agent alcalinisant et un additif de sphéronisation,
**caractérisée en ce que** la composition se présente sous la forme d'un pellet ;
dans laquelle l'agent alcalinisant est sélectionné dans le groupe comprenant le carbonate de calcium, le carbonate de magnésium, la méglumine, et un mélange de ceux-ci et dans laquelle il est utilisé en quantités allant de 0,22 à 1 partie en poids sur la base d'une partie en poids d'acide fénofibrique ;
dans laquelle l'additif de sphéronisation est choisi dans le groupe comprenant la carraghénine, la gomme de guar, l'alginate de propylène glycol, et un mélange de ceux-ci et dans laquelle il est utilisé en quantités allant de 0,05 à 0,5 % par poids sur la base du poids total du pellet ;
dans laquelle le pellet a un ratio de longueur de l'axe principal et de l'axe secondaire allant de 1,0 à 1,5 ; et
dans laquelle le pellet est en outre revêtu d'une couche de substrat de pelliculage à libération retardée.

2. La composition de la revendication 1, dans laquelle le substrat de pelliculage à libération retardée est un polymère insoluble dans l'eau.

3. La composition de la revendication 2, dans laquelle le substrat de pelliculage à libération retardée est choisi dans le groupe comprenant l'hypromellose phtalate (HPMCP), l'éthylcellulose, les copolymères poly(-éthylacrylate-méthylméthacrylate), l'acétate de cellulose et un mélange de ceux-ci.

4. La composition de la revendication 1, dans laquelle le substrat de pelliculage à libération retardée est utilisé en quantités allant de 0,05 à 0,5 parties en poids sur la base d'une partie en poids d'un pellet avant pelliculage.

5. Une méthode pour préparer la composition de la revendication 1, comprenant les étapes suivantes :
(i) d'abord le mélange au mouillé d'acide fénofibrique ou d'un sel pharmaceutiquement acceptable de celui-ci et d'un agent alcalinisant ;
(ii) l'ajout d'un excipient pharmaceutiquement acceptable au premier mélange obtenu à l'étape (i), puis un second mélange au mouillé de ceux-ci ; et
(iii) la granulation du deuxième mélange obtenu à l'étape (ii) en une forme sphérique à l'aide d'une extrudeuse et d'une machine de sphéronisation.

6. La méthode de la revendication 5, qui, à l'étape (ii), comprend en outre l'ajout d'un additif de sphéronisation au premier mélange.

7. La méthode de la revendication 5, qui comprend en outre le pelliculage du granule obtenu à l'étape (iii) avec un substrat de pelliculage à libération retardée.
